# EUROPÄISCHE PATENTSCHRIFT

(11) **EP 1 695 666 B1**
(45) Veröffentlichungstag und Bekanntmachung des Hinweises auf die Patenterteilung: **02.12.2009**
(21) Anmeldenummer: 05004048.4
(22) Anmeldetag: 24.02.2005
(51) Int. Cl.: A61B 10/00

(54) **Vorrichtung zur Durchführung eines Allergietests**
Device for allergy testing
Dispositif pour examiner une réaction allergique

(43) Veröffentlichungstag der Anmeldung: 30.08.2006
(73) Patentinhaber: Schindlbeck, Gerhard, Dr., 68519 Viernheim (DE); Bachert, Claus, Prof. Dr., 47906 Kempen (DE)
(72) Erfinder: Schindlbeck, Gerhard, Dr., 68519 Viernheim (DE); Bachert, Claus, Prof. Dr., 47906 Kempen (DE)
(74) Vertreter: Müller, Gerald Christian

(56) Entgegenhaltungen:
- EP-A- 0 026 501
- WO-A-88/09149
- WO-A-99/08601
- DE-A1- 2 444 379
- DE-C1- 4 328 112

## Beschreibung

### I. Technisches Gebiet

Die vorliegende Erfindung bezieht sich auf eine Vorrichtung zur Durchführung eines Allergietests, bei dem die Haut eines Testlebewesens, insbesondere einer Testperson, mit Allergenen in Berührung gebracht wird und vor, nach oder während des Aufbringens der Allergene auf die Haut letztere geritzt bzw. verletzt wird, um eine Reaktion des Testlebewesens auf die Allergene hervorzurufen.

### II. Technischer Hintergrund

Aus der DE 43 28 112 C1 ist bereits eine Vorrichtung zur Durchführung eines Allergietests der in Rede stehenden Art bekannt. Die bekannte Vorrichtung weist eine auf die Haut auflegbare Abdeckeinheit und eine mit dieser gelenkig verbundene Behältereinheit mit mehreren Behältern zur Aufnahme der Allergene auf. Nach dem Auflegen der Abdeckeinheit beispielsweise auf den Unterarm einer Testperson wird die Haut mit Hilfe eines Ritz- bzw. Prickwerkzeuges durch die Durchbrüche in der Abdeckeinheit hindurch geritzt bzw. verletzt. Anschließend kann eine die Behälter der Behältereinheit verschließende Schließfolie abgezogen und die Behältereinheit auf die Abdeckeinheit geklappt werden. Dadurch gelangen die in den Behältern befindlichen Allergene durch die Durchbrüche hindurch auf die verletzten Hautstellen, so dass die Reaktionen der Testperson auf die diversen Allergene abgewartet werden können.

Für den Fall, dass die bekannte Vorrichtung nach dem Aufbringen der Allergene auf die Haut und dem Ritzen derselben entfernt werden soll, bevor etwaige allergische Reaktionen beobachtet werden können, weist die Vorrichtung eine auf die Haut übergabefähige Kennzeichnung zur Zuordnung bestimmter Allergene zu bestimmten Allergieteststellen auf. Bei dieser übergabefähigen Kennzeichnung handelt es sich entweder um übertragbare und auf der Haut gut haftende Farbe oder um eine übertragende Folie ähnlich einem Abziehbild. Übergabefähige Kennzeichnungen dieser Art weisen den Nachteil auf, dass sie einerseits unleserlich übertragen werden können oder verwischen und andererseits in Abhängigkeit von ihrer Hauthaftung häufig länger als notwendig auf der Haut verbleiben, was aus ästhetischen Gründen von vielen Testpersonen nicht gewünscht ist.

In Zusammenhang mit der Vorrichtung gemäß DE 43 28 112 C1 ist es auch bekannt, die Behältereinheit nach dem Ritzen der Allergieteststellen auf der Haut an der Abdeckeinheit zu fixieren. Dieses Fixieren erfolgt entweder durch partielles Verkleben oder mittels eines Klettbandstreifens, der beispielsweise um den Arm der Testperson herumgeführt wird. Ein Fixieren dieser Art ist für die den Allergietest durchführende Person verhältnismäßig aufwändig, da zum Einen Hilfsmittel wie Klebstoff oder Klettbandstreifen bereitgehalten und zum Anderen mehrere Handgriffe durchgeführt werden müssen. Dieser Aufwand wirkt sich nachteilig sowohl auf die Kosten des Allergietests als auch auf die für seine Durchführung erforderliche Zeit aus.

### III. Darstellung der Erfindung

### a) Technische Aufgabe

Es ist daher die Aufgabe der vorliegenden Erfindung, eine Vorrichtung zur Durchführung eines Allergietests zu schaffen, die das Fixieren einer Behältereinheit auf einer Abdeckeinheit auf möglichst einfache Art und Weise erlaubt.

### b) Lösung der Aufgabe

Diese Aufgabe wird mit einer Vorrichtung mit den Merkmalen des Anspruchs 1 gelöst. Weitere Ausgestaltungsformen der vorliegenden Erfindung ergeben sich aus den Unteransprüchen.

Erfindungsgemäß wird eine Vorrichtung zur Durchführung eines Allergietests vorgeschlagen, die eine Behältereinheit mit mehreren Behältern zur Aufnahme von Allergenen und eine Abdeckeinheit mit mehreren Durchbrüchen zur Abgrenzung von Allergieteststellen umfasst und bei der auf derjenigen Seite der Abdeckeinheit, auf die die Behältereinheit auflegbar ist, zumindest teilweise ein Klebefilm vorgesehen ist, der von einer Abziehfolie bedeckt ist, die mit den Durchbrüchen in der Abdeckeinheit im Wesentlichen fluchtende Durchgangslöcher aufweist. Der Vorteil einer derartigen Vorrichtung besteht darin, dass durch einen einzigen Handhabungsschritt, nämlich das Entfernen der Abziehfolie, gewährleistet ist, dass die Behältereinheit beim Auflegen auf die Abdeckeinheit sicher und unverrutschbar auf letzterer fixiert wird. Ein Verlaufen oder Verwischen von Allergenen zwischen Abdeckeinheit und Behältereinheit wird vermieden. Darüber hinaus können Behältereinheit und Abdeckeinheit nach ausreichender Einwirkdauer als eine zusammenklebende, kompakte Einheit von der Allergieteststelle entfernt und entsorgt werden.

Vorzugsweise liegen die Querschnitte der Durchgangslöcher in der Abziehfolie vollständig innerhalb der Querschnitte der Durchbrüche in der Abdeckeinheit. Des weiteren können die Durchgangslöcher und die Durchbrüche dieselbe Querschnittsform und denselben Querschnittsmittelpunkt aufweisen. Sind dann zusätzlich noch die Querschnittsflächen der Durchgangslöcher kleiner als die Querschnittsflächen der Durchbrüche, so ergibt sich der Vorteil, dass die Haut beim Ritzen mit einem Prickwerkzeug durch die Durchgangslöcher und die Durchbrüche hindurch immer in einem Hautbereich geritzt wird, der bezüglich der Durchgangslöcher in der Abdeckeinheit zentriert liegt. Dadurch wird das Überlappen allergischer Reaktionen bei benachbarten Allergieteststellen unwahrscheinlicher.

Die Querschnitte der Durchgangslöcher und/oder der Durchbrüche können beispielsweise kreisförmig, rechteckförmig oder quadratisch ausgebildet werden. Dabei müssen Durchgangslöcher und Durchbrüche nicht zwingend identische Querschnittsform aufweisen. Je nach Bedarf sind auch Kombinationen verschiedener Querschnittsgeometrien denkbar. Vorteilhaft ist es, die Durchgangslöcher in der Abziehfolie kreuzförmig auszubilden. Dies ermöglicht ein kreuzweises Ritzen der Allergieteststellen auf der Haut, so dass die Haut zumindest in dem Kreuzmittelpunkt zuverlässig geritzt wird.

In besonders vorteilhafter Weise können die Allergieteststellen voneinander abgedichtet bzw. isoliert werden, wenn jeder Behälter der Behältereinheit in seinem Randbereich eine Dichtlippe aufweist. Vorzugsweise durchgreifen die Dichtlippen bei auf die Abdeckeinheit aufgelegter Behältereinheit den jeweiligen Durchbruch, so dass die Dichtlippen jeweils in Kontakt mit der Haut um die jeweilige Allergieteststelle herum gebracht werden können. Dadurch werden die Allergieteststellen wirksam voneinander abgedichtet, so dass ein einer Teststelle zugeordnetes Allergen nicht zu einer anderen Allergieteststelle gelangen kann. Die erfindungsgemäße Dichtlippe kann in besonders vorteilhafter Weise beispielsweise beim Heißsiegelverkleben einer die Behälter verschließenden Schließfolie mit der Behältereinheit erzeugt werden.

Die Vorrichtung zur Durchführung eines Allergietests kann wenigstens eine Kennzeichnungseinheit aufweisen, die lösbar an der Vorrichtung befestigt ist und die auf ihrer bei Durchführung des Allergietests der Haut zugewandten Seite zumindest teilweise mit einem Haftmittel versehen ist, so dass die Vorrichtung von der Haut eines Testlebewesens entfernt werden kann und gleichzeitig die wenigstens eine Kennzeichnungseinheit auf der Haut haften bleibt. Das Trennen der Kennzeichnungseinheit von dem Rest der Vorrichtung erfolgt dabei während des Entfernens der Vorrichtung von der Haut.

In an sich bekannter Weise ist die Behältereinheit der erfindungsgemäßen Vorrichtung vorzugsweise flach ausgebildet und besteht ebenso vorzugsweise aus einem dünnen, flexiblen Material, wie beispielsweise Kunststoff, das an die Kontur der Allergieteststelle, wie etwa dem Unterarm einer Testperson, anpassbar ist. Die Kennzeichnungseinheit kann aus demselben Material wie die Behältereinheit bestehen und beispielsweise entlang einer perforierten Reißlinie an einer Seitenkante der Behältereinheit befestigt sein. Dies bringt den Vorteil mit sich, dass Behältereinheit und Kennzeichnungseinheit in ein und demselben Fertigungsschritt hergestellt werden können. Nach dem Entfernen der Behältereinheit von der Allergieteststelle verbleibt die wenigstens eine Kennzeichnungseinheit ähnlich wie ein Klebestreifen auf der Haut der Testperson, so dass anhand der Informationen, die auf der von der Haut abgewandten Seite der Kennzeichnungseinheit aufgebracht sind, eine eindeutige Zuordnung eines bestimmten Allergens zu einer bestimmten Allergieteststelle zum Zwecke der Auswertung des Allergietests erfolgen kann. Dementsprechend werden die Zuordnungsinformationen nicht mehr mittels Farbe oder in der Art eines Abziehbildes direkt auf die Haut aufgebracht. Die in der Art eines Klebestreifens ausgebildete Kennzeichnungseinheit haftet lediglich auf der Haut des Testlebewesens und kann nach Abschluss der Allergietestauswertung problemlos von der Haut abgezogen werden.

Zusätzlich zu der Behältereinheit weist die Vorrichtung eine Abdeckeinheit mit mehreren Durchbrüchen zur Abgrenzung der Allergieteststellen aufweist. Dabei ist die Abdeckeinheit in an sich bekannter Weise auf die Haut des Testlebewesens auflegbar und die Behältereinheit ist ihrerseits derart auf die Abdeckeinheit auflegbar, dass die Durchbrüche mit den Behältern im Wesentlichen fluchten. Die Kennzeichnungseinheit kann bei Vorrichtungen dieser Art entweder an einer Seitenkante der Behältereinheit oder an einer Seitenkante der Abdeckeinheit lösbar befestigt sein. Da Behältereinheit und Abdeckeinheit in der Aufsicht vorzugsweise dieselbe Geometrie aufweisen, ragt die Kennzeichnungseinheit in Abhängigkeit davon, ob sie an der Abdeckeinheit oder an der Behältereinheit befestigt ist, seitlich über die Behältereinheit oder die Abdeckeinheit hinaus. Bei an der Behältereinheit angeordneter Kennzeichnungseinheit wird der besondere Vorteil erreicht, dass die erfindungsgemäße Vorrichtung aufgrund der klebebandartigen Niederhaltewirkung der Kennzeichnungseinheit in durchgehend flächigem Kontakt auf der meist gekrümmten Hautoberfläche der Allergieteststelle aufliegt.

Die erfindungsgemäße Kennzeichnungseinheit liegt in ihrer von der Behältereinheit oder der Abdeckeinheit abragenden Stellung vorzugsweise in der von der Behältereinheit oder der Abdeckeinheit aufgespannten Ebene. Zur Minimierung der Größe der erfindungsgemäßen Vorrichtung und somit des Platzbedarfs während Transport und Lagerung ist es denkbar, die Kennzeichnungseinheit zunächst in einem auf die Behältereinheit oder auf die Abdeckeinheit geklappten Zustand anzuordnen. Soll die Vorrichtung schließlich zur Durchführung eines Allergietests verwendet werden, so wird die Kennzeichnungseinheit um im Wesentlichen 180° um diejenige Seitenkante, an der sie befestigt ist, in ihre von der Behältereinheit oder der Abdeckeinheit abragende Stellung geschwenkt bzw. geklappt.

Insbesondere wenn in an sich bekannter Weise mehrere im Wesentlichen parallele Reihen von Behältern oder von Behältern und Durchbrüchen vorgesehen sind, ist vorzugsweise an zwei gegenüber liegenden Seitenkanten der Behältereinheit oder der Abdeckeinheit jeweils eine Kennzeichnungseinheit angeordnet. Die Zuordnungsinformationen der jeweiligen Kennzeichnungseinheit betreffen dann diejenige Reihe von Behältern bzw. Durchbrüchen bzw. Allergieteststellen, die der Kennzeichnungseinheit am Nächsten liegt.

Denkbar ist auch, nicht nur die Kennzeichnungseinheit lösbar an der Behältereinheit oder der Abdeckeinheit anzuordnen, sondern zusätzlich die Behälter aus der Behältereinheit herauslösbar auszubilden. Bei dieser Variante kann die Behältereinheit auf die Haut aufgelegt bzw. aufgeklebt und anschließend die Haut mit Hilfe eines Prickwerkzeuges durch die Behälter hindurch geritzt werden. Das Abziehen der Behältereinheit von der Haut erfolgt derart, dass sowohl die einzelnen Behälter der Behältereinheit als auch die Kennzeichnungseinheit auf der Haut haften bleiben. Nach einer gewissen Einwirkdauer können schließlich die einzelnen Behälter entfernt und die Auswertung des Allergietests mit Hilfe der Zuordnungsinformationen auf der Kennzeichnungseinheit durchgeführt werden.

Die erfindungsgemäße Vorrichtung kann auch ein Rahmenteil mit einer Rahmenöffnung aufweisen, die bei auf die Haut aufgelegter Vorrichtung das Arbeitsfeld, in dem sich die Allergieteststellen befinden, definiert. Dabei sind die Behältereinheit, die Abdeckeinheit und die wenigstens eine Kennzeichnungseinheit jeweils an einer äußeren Seitenkante des Rahmenteils befestigt. Insbesondere zur Minimierung der Größe der Vorrichtung und somit des Platzbedarfs bei Transport und Lagerung können die Behältereinheit und die Abdeckeinheit gelenkig an dem Rahmenteil befestigt sein, so dass sie um im Wesentlichen 180° in die Rahmenöffnung klapp- bzw. schwenkbar sind.

Wie bei der Variante ohne Rahmenteil kann die Kennzeichnungseinheit auch bei der Variante mit Rahmenteil entlang einer perforierten Reißlinie lösbar befestigt sein, allerdings nicht an der Behältereinheit und nicht an der Abdeckeinheit, sondern an dem Rahmenteil. Die Behältereinheit und die Abdeckeinheit sind vorzugsweise an gegenüberliegenden Seiten des Rahmenteils befestigt. Die Kennzeichnungseinheit kann um im Wesentlichen 180° zwischen einer von der Seitenkante des Rahmenteils abragenden Stellung in eine im Wesentlichen in der Rahmenöffnung liegende Stellung verschwenkt werden. Insbesondere bei im Wesentlichen parallelen Reihen von Behältern und Durchbrüchen befindet sich die Kenzeichnungseinheit vorzugsweise an zwei gegenüberliegenden Seitenkanten des Rahmenteils.

Bei dem auf der der Haut zugewandten Seite der Kennzeichnungseinheit angebrachten Haftmittel kann es sich um einen Klebefilm handeln, der zum Schutz von Verunreinigungen mit einer entfernbaren Schutzfolie bedeckt ist. Vorzugsweise ist die gesamte der Haut zugewandte Seite der Kennzeichnungseinheit mit dem Klebefilm überzogen. Denkbar ist jedoch auch ein nur teil- oder stellenweiser Überzug, solange ein sicheres Haften auf der Haut des Testlebewesens gewährleistet ist.

### c) Ausführungsbeispiele

Nachfolgend werden mehrere Ausführungsformen der vorliegenden Erfindung beispielhaft anhand der beigefügten Zeichnungen beschrieben. Es zeigen:
- Fig. 1:: eine perspektivische Ansicht einer ersten Ausführungsform der erfindungsgemäßen Vorrichtung;
- Fig. 2:: eine Schnittansicht gemäß Schnitt A-A in Fig. 1;
- Fig**.** 3:: eine Schnittansicht gemäß Schnitt B-B in Fig. 1;
- Fig. 4:: eine Aufsicht auf die Vorderseite der Vorrichtung gemäß Fig. 1 ohne Schließ- und Abziehfolie;
- Fig. 5:: eine Aufsicht auf die Rückseite der Vorrichtung gemäß Fig. 1; und
- Fig. 6:: eine Aufsicht auf eine zweite Ausführungsform der erfindungsgemäßen Vorrichtung.
In den Zeichnungen sind gleiche Teile, die gleiche Funktionen haben, mit denselben Bezugszeichen gekennzeichnet.

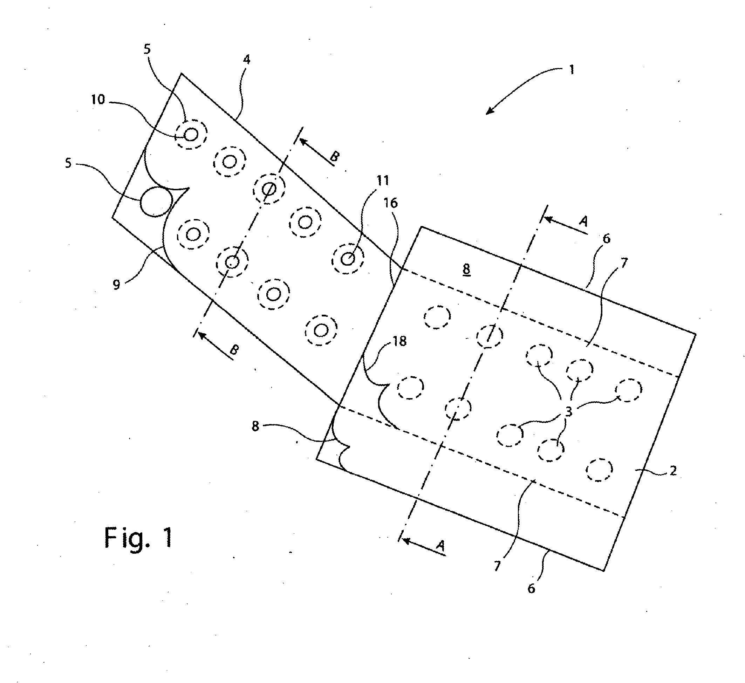

In Fig. 1 ist eine erste Ausführungsform der erfindungsgemäßen Vorrichtung 1 dargestellt. Die Vorrichtung 1 besteht aus einer flachen, rechteckförmigen, als Kammerteil fungierenden Behältereinheit 2, einer flachen, rechteckförmigen, als Schablonenteil fungierenden Abdeckeinheit 4 sowie zwei flachen, rechteckförmigen Kennzeichnungseinheiten 6, die auf den gegenüber liegenden langen Seiten der Abdeckeinheit 4 flügelartig angeordnet sind. Die Behältereinheit 2, die Abdeckeinheit 4 und die Kennzeichnungseinheiten 6 sind flexibel und biegsam ausgestaltet, so dass sie an Krümmungen oder Wölbungen der Hautoberfläche angepasst werden können. Bei der gezeigten Ausführungsform bestehen die vorgenannten Einheiten aus einem dünnen Kunststoff, der ggf. in geeigneter Weise beschichtet ist.

In an sich bekannter Weise ist die Behältereinheit 2 mit Hilfe eines Gelenks 16 klapp- oder drehbar mit der Abdeckeinheit 4 verbunden. Bei auf die Abdeckeinheit 4 geklappter Behältereinheit 2 ragen somit die beiden Kennzeichnungseinheiten 6 über die langen Seiten der Abdeckeinheit 4 hinaus und können in Kontakt mit der Haut gebracht werden. Die Kennzeichnungseinheiten 6 sind jeweils lösbar entlang einer perforierten Reißlinie 7 an der Behältereinheit 2 befestigt. Alternativ kann die Lösbarkeit durch eine Materialverdünnung zwischen Kennzeichnungseinheiten 6 und Behältereinheit 2 bewirkt werden. Zusätzlich können die Reißlinien 7 als Gelenke fungieren, so dass die Kennzeichnungseinheiten 6 von der in den Fig. 1, 4 und 5 gezeigten Stellung um ca. 180° auf die Behältereinheit 2 geklappt werden können. Dabei ist ein Klappen auf die in Fig. 1 sichtbare Vorderseite oder auf die in Fig. 1 nicht sichtbare Rückseite der Behältereinheit 2 denkbar. Vorzugsweise erfolgt das Einklappen der Kennzeichnungseinheiten 6 auf die in Fig. 1 gezeigte Vorderseite, da in diesem Fall die Kennzeichnungseinheiten 6 bei auf die Abdeckeinheit 4 geklappter Behältereinheit 2 zwischen Abdeckeinheit 4 und Behältereinheit 2 verstaut werden können. Unabhängig von der Klapprichtung der Kennzeichnungseinheiten 6 wird durch das Einklappen derselben ein Platzspareffekt bewirkt, der dazu führt, dass die erfindungsgemäße Vorrichtung 1 nicht mehr Platz benötigt als die aus dem Stand der Technik bekannten Vorrichtungen zur Durchführung von Allergietests.

Die Behältereinheit 2 weist in an sich bekannter Weise Behälter 3 zur Aufnahme von Allergenen auf. Wie in den Fig. 1, 4 und 5 zu erkennen ist, sind insgesamt zwei parallele Reihen mit jeweils fünf Behältern 3 vorgesehen. Denkbar sind jedoch auch beliebige andere Geometrien von Behälteranordnungen, sofern sie den Erfordernissen der Durchführung des Allergietests Rechnung tragen. Beispiele hierfür sind Behälteranordnungen, deren Geometrien in der Aufsicht Tier- bzw. Comicfigurenumrisse oder eine Herzform widerspiegeln. Behälteranordnungen dieser Art können Kindern ggf. eine etwaige Angst vor dem Allergietest nehmen. Außerdem können im Übrigen beliebige Formen, Farben und Anzahlen von in der Behältereinheit 2 ausgebildeten Behältern 3 vorgesehen werden.

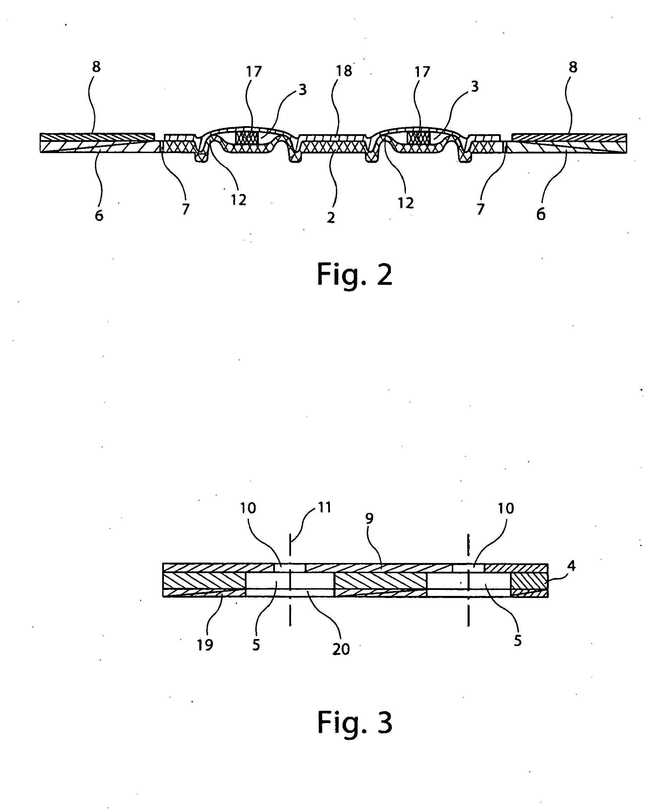

Fig. 2 zeigt die Schnittansicht gemäß Schnitt A-A in Fig. 1 durch zwei Behälter 3. Bei der gezeigten Ausführungsform befindet sich in den Behältern 3 jeweils ein mit einem bestimmten Allergen getränktes vliesartiges Schwammgewebe 17. Anstatt in flüssiger Form können die Allergene allerdings auch ohne Schwammgewebe 17 in fester, gelartiger oder pastenartiger Form in den Behältern 3 angeordnet sein. Darüber hinaus ist alternativ denkbar, in den Behältern 3 eine Pricknadel, an der sich ein Allergen befindet, anzuordnen. Ein Verletzen der Haut ist dann durch Drücken auf den jeweiligen Behälter 3 von der in Fig. 2 unteren Seite auf die Behältereinheit möglich. Die Behälter 3 sind bei der gezeigten Ausführungsform mittels einer vorzugsweise aus Aluminium bestehenden Schließfolie 18 verschlossen. Die Schließfolie 18 sorgt dafür, dass die in den Schwammgeweben 17 enthaltenen Allergene haltbar bleiben und möglichst lange für die Zwecke eines Allergietests verwendet werden können. Zum Öffnen der Behälter 3 kann die Folie von Hand von der Behältereinheit 2 abgezogen werden. Zu diesem Zweck kann die Schließfolie 18 eine Grundfläche aufweisen, die geringfügig größer als diejenige der Behältereinheit 2 ist. Der über die Seitenränder der Behältereinheit 2 hinaus stehende Teil der Schließfolie 18 kann dann als Lasche zum Abziehen der Schutzfolie 18 verwendet werden.

In Fig. 2 sind auch die beiden Kennzeichnungseinheiten 6 zu erkennen, die über die perforierte Reißlinie 7 mit der Behältereinheit 2 verbunden sind. Auf der in Fig. 2 oberen Seite der Kennzeichnungseinheiten 6 befindet sich jeweils eine abziehbare Schutzfolie 8, die einen auf der in Fig. 2 oberen Seite der Kennzeichnungseinheiten 6 jeweils befindlichen Klebefilm abdeckt. Dieser Klebefilm ermöglicht ein Fixieren der Kennzeichnungseinheiten 6 auf der Haut eines Testlebewesens, insbesondere am Unterarm einer Testperson.

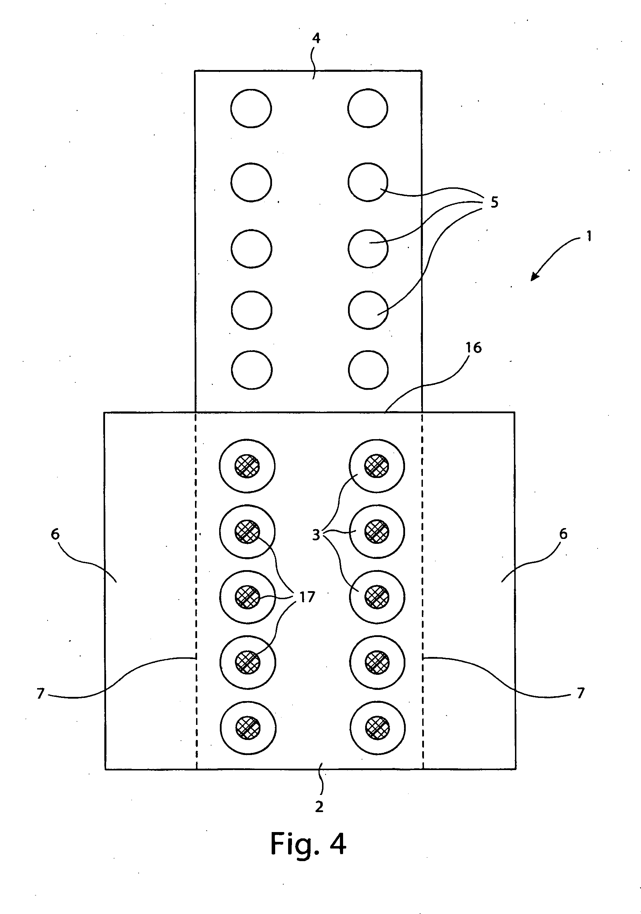

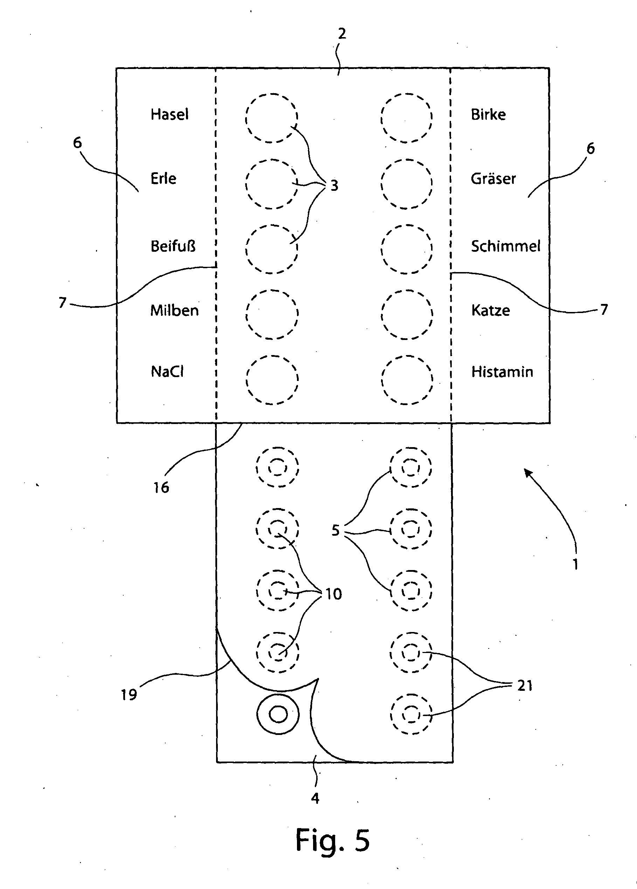

Auf der in Fig. 2 unteren Seite der Kennzeichnungseinheiten 6, d.h. auf der in den Fig. 1 und 4 nicht sichtbaren Rückseite, die in Fig. 5 zu sehen ist, befinden sich erfindungsgemäß jeweils Zuordnungsinformationen, die Auskunft darüber geben, welche Art von Allergen in einem bestimmten Behälter 3 enthalten ist. Wie in Fig. 5 zu erkennen, sind die Zuordnungsinformationen zu diesem Zweck derart auf die Kennzeichnungseinheiten 6 aufgebracht, dass sie sich unmittelbar neben dem jeweiligen Behälter 3 befinden, dessen Allergen sie kennzeichnen sollen. Bei dem gezeigten Ausführungsbeispiel sind die Zuordnungsinformationen jeweils als Wort aufgebracht, das die Allergie wiedergibt, auf die das in dem jeweiligen Behälter 3 befindliche Allergen die Testperson testet. Wie in Fig. 5 zu erkennen, handelt es sich um die Worte "Hasel" "Erle", "Beifuß", "Milben", "NaCl", "Birke", "Gräser", "Schimmel", "Katze" und "Histamin". Die Zuordnungsinformationen können im Rahmen der vorliegenden Erfindung natürlich auch von anderen Allergenangaben in Text- und/oder Symbolform gebildet werden.

Fig**.** 3 zeigt den Schnitt B-B gemäß Fig. 1 durch die Abdeckeinheit 4. Die Abdeckeinheit 4 weist Durchbrüche 5 auf, die bei auf die Abdeckeinheit 4 geklappter Behältereinheit 2 in an sich bekannter Weise mit den Behältern 3 im Wesentlichen fluchten. Auf der in Fig. 3 oberen Seite der Abdeckeinheit 4, auf die auch in Fig. 1 geblickt wird, befindet sich eine Abziehfolie 9, die einen auf der in Fig. 3 oberen Seite der Abdeckeinheit 4 befindlichen Klebefilm bedeckt. Dieser Klebefilm kann im Wesentlichen auf der gesamten oder nur auf einem Teil der Oberfläche der Abdeckeinheit 4 aufgebracht sein. Beispielsweise ist denkbar, dass nur einige Klebefilmpunkte vorgesehen sind. Der von der Abziehfolie 9 bedeckte Klebefilm ermöglicht ein sicheres Fixieren der auf die Abdeckeinheit 4 geklappten Behältereinheit 2 an bzw. auf der Abdeckeinheit 4.

Darüber hinaus weist die Abziehfolie 9 Durchgangslöcher 10 auf, die jeweils mit den Durchbrüchen 5 in der Abdeckeinheit 4 fluchten und dabei - in Fig. 3 von oben betrachtet - mit ihrer Querschnittsform vollständig innerhalb der Querschnittsform des jeweiligen Durchbruchs 5 liegen. Der besondere Vorteil dieser Ausgestaltung wird in Zusammenhang mit der Funktionsweise der Vorrichtung 1 weiter unten beschrieben. Bei der gezeigten Ausführungsform weisen sowohl die Durchbrüche 5 als auch die Durchgangslöcher 10 eine kreisrunde Geometrie auf, wobei die jeweiligen Kreismittelpunkte 11 aufeinander fallen. Denkbar sind hier auch andere Querschnittsgeometrien, wie beispielsweise quadratische, rechteckige, elliptische oder kreuzförmige Formen. Auch voneinander abweichende Querschnittsgeometrien für die Durchbrüche 5 einerseits und die Durchgangslöcher 10 andererseits sind denkbar.

Auf der in Fig. 3 unteren Seite der Abdeckeinheit 4 ist eine weitere Abziehfolie 19 angebracht, die einen weiteren, auf der in Fig. 3 unteren Seite der Abdeckeinheit 4 befindlichen Klebefilm bedeckt. Dieser zumindest teilweise aufgebrachte Klebefilm ermöglicht das sichere Befestigen bzw. Fixieren der Abdeckeinheit 4 auf der Hautoberfläche der Testperson. Die Abziehfolie 19 weist ihrerseits Durchgangsöffnungen 20 auf, die bei der gezeigten Ausführungsform nach Anzahl, Lage, Querschnittsgeometrie und -größe den Durchbrüchen 5 entsprechen. Denkbar ist auch, die Durchgangsöffnungen 20 mit anderen Querschnittsgeometrien und - größen auszubilden oder die Abziehfolie 19 vollständig ohne Durchgangsöffnungen 20 auszubilden, da die Abziehfolie 19 vor Durchführung des eigentlichen Allergietests, insbesondere des Hautritzens, vollständig von der Abdeckeinheit 4 entfernt wird.

Fig. 4 zeigt eine Aufsicht auf die in Fig. 1 zu erkennende Vorderseite der erfindungsgemäßen Vorrichtung 1, wobei von der Behältereinheit 3 die Schließfolie 18, von den Kennzeichnungseinheiten 6 die Schutzfolien 8 und von der Abdeckeinheit 4 die Abziehfolie 9 entfernt wurden. Dementsprechend sind in Fig. 4 die offenen Behälter 3 mit den darin befindlichen Schwammgeweben 17 zu erkennen. Des weiteren sind in der Abdeckeinheit 4 die Durchbrüche 5 zu erkennen, deren äußere Randbereiche nicht mehr von der Abziehfolie 9 bedeckt sind.

Fig. 5 zeigt eine Ansicht auf die in den Fig. 1 und 4 nicht sichtbare Rückseite der erfindungsgemäßen Vorrichtung 1. Wie in Fig. 4 liegen auch hier die Behältereinheit 2 und die Abdeckeinheit 4 in einer Ebene. Die in der Behältereinheit 2 angeordneten Behälter 3 sind in Fig. 5 nur von ihrer verschlossenen Rückseite her sichtbar. Auf den beiden Kennzeichnungseinheiten 6 sind die Zuordnungsinformationen "Hasel", "Erle", "Beifuß", "Milben", "NaCl", "Birke", "Gräser", "Schimmel", "Katze" und "Histamin" sichtbar. Durch die in der Abdeckeinheit 4 vorgesehenen Durchbrüche 5 hindurch sind kreisringförmige Bereiche 21 der Abziehfolie 9 sichtbar.

Nachfolgend wird nun beispielhaft die Funktions- und Anwendungsweise der Vorrichtung 1 gemäß der Fig. 1-5 im Rahmen der Durchführung eines Allergietests beschrieben:

Nach Vorbereitung einer Hautoberfläche einer Testperson, beispielsweise am Unterarm, durch Reinigen derselben wird die Abziehfolie 19 von der Abdeckeinheit 4 entfernt und letztere mit Hilfe des freigelegten Klebefilms durch Andrücken auf der Hautoberfläche befestigt. Anschließend wird mit Hilfe eines an sich bekannten Prick- oder Ritzwerkzeugs die Hautoberfläche durch die in der Abziehfolie 9 befindlichen Durchgangslöcher 10 hindurch geritzt. Hierzu kann mit dem Prickwerkzeug beispielsweise jeweils einmal geradlinig und ohne Absetzen über die in Fig. 1 gezeigten Reihen von Durchgangslöchern 10 gefahren werden. Nach dem Ritzen der Hautoberfläche wird die Abziehfolie 9 entfernt, um den darunter liegenden Klebefilm freizulegen. Des Weiteren werden die Behälter 3 durch Abziehen der Schließfolie 18 geöffnet und die Klebefilme auf den Kennzeichnungseinheiten 6 durch Entfernen der Schutzfolien 8 freigelegt. Daraufhin kann die Behältereinheit 2 samt Kennzeichnungseinheiten 6 um ca. 180° um das Gelenk 16 derart geklappt werden, dass die Behälter 3 mit den Durchbrüchen 5 im Wesentlichen fluchten und die Allergene in den Schwammgeweben 17 auf die Allergieteststellen gelangen, die von den Durchbrüchen 5 auf der Hautoberfläche abgegrenzt werden.

Aufgrund des Klebefilms auf der von der Haut abgewandten Seite der Abdeckeinheit 4 haftet die Behältereinheit sicher und unverrutschbar auf der Abdeckeinheit 4. Behältereinheit 2 und Abdeckeinheit 4 bilden eine kompakt handhabbare Einheit, die nicht mehr nur linienförmig über das Gelenk 16, sondern flächig miteinander verbunden ist. Darüber hinaus werden die beiden Kennzeichnungseinheiten 6 auf dem Unterarm der Testperson festgedrückt, so dass sie an der Hautoberfläche haften.

Nach einer gewissen Einwirkdauer auf die Allergieteststelleri kann die kompakt handhabbare Einheit aus Behältereinheit 2 und Abdeckeinheit 4 von der Hautoberfläche entfernt werden, indem sie ergriffen und bei gleichzeitigem Andrücken bzw. Festhalten der Kennzeichnungseinheiten 6 an der Hautoberfläche entfernt wird. Dabei wird die Behältereinheit 2 entlang der perforierten Reißlinien 7 von den Kennzeichnungseinheiten 6 abgerissen, die zur Auswertung des Allergietests auf der Hautoberfläche der Testperson verbleiben. Die Reaktionen der Testperson auf die einzelnen Allergene kann dann von der den Test durchführenden Person abgelesen und mit Hilfe der auf den Kennzeichnungseinheiten 6 befindlichen Zuordnungsinformationen dokumentiert werden. Nach Beendigung der Dokumentation des Testergebnisses können die Kennzeichnungseinheiten 6 von der Hautoberfläche abgezogen werden, so dass keine ggf. erst nach längerer Zeit vollständig entfernbaren Kennzeichnungen mehr am Unterarm der Testperson zurückbleiben. Bei Bedarf können sogar die Kennzeichnungseinheiten 6 durch Einkleben oder sonstiges Ablegen in einer ärztlichen Akte im Rahmen der Dokumentation verwendet werden.

Beim Ritzen der Haut mit dem Prickwerkzeug bringen die in der Abziehfolie 9 vorhandenen Durchgangslöcher 10 einen besonderen Vorteil mit sich. Die Ritzung der Haut erfolgt im Hinblick auf die größeren Durchbrüche 5 im Wesentlichen zentriert. Dadurch werden Hautritzungen am Rand der Durchbrüche 5 vermieden, so dass ein unerwünschtes Annähem zweier geritzter Allergieteststellen an einander unmöglich ist. In besonders vorteilhafter Weise kann auch die Zuverlässigkeit der Hautritzung gesteigert werden. Hierzu ist denkbar, nicht kreisrunde Durchgangslöcher 10, sondern kreuzförmige Durchgangslöcher 10 auszubilden. Das Ritzen der Haut kann dann erfolgen, indem das Prickwerkzeug in zwei im Wesentlichen senkrecht zueinander verlaufenden Ritzgeraden über das jeweils kreuzförmige Durchgangsloch 10 geführt wird. Zumindest im Schnittpunkt der beiden Ritzgeraden wird die Hautoberfläche zuverlässig geritzt.

Um zuverlässig auszuschließen, dass das einer bestimmten Allergieteststelle zugeordnete Allergen zwischen Hautoberfläche und Abdeckeinheit 4 zu einer anderen Allergieteststelle gelangt, was das Ergebnis des Allergietests naturgemäß verfälschen würde, weist die Behältereinheit 2 eine in Fig. 2 zu erkennende Dichtlippe 12 auf. Die Dichtlippe 12 liegt im Randbereich eines jeden Behälters 3 und bildet vorzugsweise zusätzlich dessen Seitenwandung. Sie kann in der Aufsicht entweder dieselbe Geometrie aufweisen wie die Durchbrüche 5 in der Abdeckeinheit 4 oder bei Bedarf mit einer davon abweichenden Geometrie ausgebildet sein. Wie in Fig**.** 2 zu erkennen ist, steht die Dichtlippe 12 in Fig. 2 nach oben über die Oberfläche der Behältereinheit 2 hervor, so dass sie beim Zusammenlegen von Behältereinheit 2 und Abdeckeinheit 4 durch die in letzterer vorhandenen Durchbrüche 5 hindurch greift und in Kontakt mit der Hautoberfläche treten kann. Dadurch wird die jeweilige Allergieteststelle durch Berührung zwischen Dichtlippe 12 und Hautoberfläche von anderen Allergieteststellen sicher isoliert. Bei der gezeigten Ausführungsform wird die Dichtlippe 12 in besonders vorteilhafter Weise gleichzeitig mit dem Heißsiegelverkleben der Schließfolie 18 an der Behältereinheit 2 hergestellt. Dabei wird in Fig. 2 von oben ein Heißsiegelstempel mit hier kreisförmiger Stempelfläche an die Schließfolie 18 herangeführt, der letztere mit der Behältereinheit 2 verpresst. Dabei ergeben sich Materialverdrängungen insbesondere an der Behältereinheit 2, die zur Ausbildung der in Fig. 2 gezeigten Dichtlippe 12 führen.

Im Sinne der vorliegenden Erfindung ist es alternativ möglich, die Kennzeichnungseinheiten 6 nicht an der Behältereinheit 2, sondern stattdessen an den langen Seiten der Abdeckeinheit 4 anzuordnen. Die voranstehend beschriebene Funktionsweise der Vorrichtung 1 ändert sich dadurch im Wesentlichen nicht. Vor dem Auflegen der Abdeckeinheit 4 auf die Hautoberfläche sind zusätzlich zu der Abziehfolie 19 lediglich noch die Schutzfolien 8 von den Kennzeichnungseinheiten 6 abzuziehen.

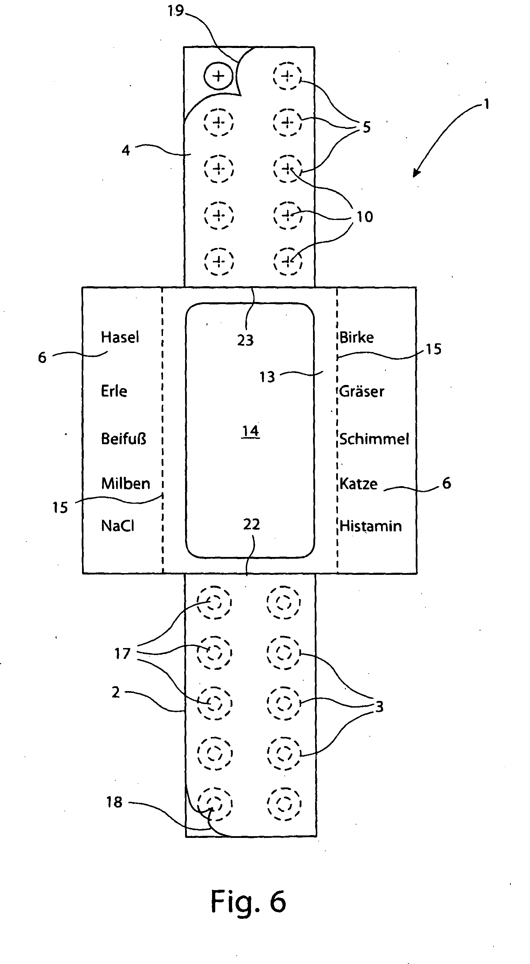

Fig. 6 zeigt eine zweite Ausführungsform einer erfindungsgemäßen Vorrichtung 1. Im Gegensatz zu der ersten Ausführungsform weist die zweite Ausführungsform ein flaches, im Wesentlichen rechteckförmiges Rahmenteil 13 auf, das eine Rahmenöffnung 14 umschließt. An den beiden gegenüberliegenden, langen Seiten des Rahmenteils 13 ist jeweils über eine perforierte Reißlinie 15 eine erfindungsgemäße Kennzeichnungseinheit 6 angeordnet. An der in Fig. 6 unteren, kurzen Seite des Rahmenteils 13 ist mittels eines Gelenks 22 eine Behältereinheit 2 befestigt, die ihrem Aufbau nach derjenigen entspricht, die in Zusammenhang mit der ersten Ausführungsform beschrieben wurde. An der in Fig. 6 oberen, kurzen Seite des Rahmenteils 13 ist mittels eines Gelenks 23 eine Abdeckeinheit 4 befestigt, die derjenigen entspricht, die in Zusammenhang mit der ersten Ausführungsform beschrieben wurde.

Nachfolgend werden beispielhaft die einzelnen Arbeitsschritte zur Durchführung eines Allergietests mittels der Ausführungsform gemäß Fig. 6 beschrieben:

Zunächst werden die Schutzfolien 8 von den in Fig. 6 nicht zu sehenden Seiten der beiden Kennzeichnungseinheiten 6 entfernt. Mit Hilfe der dadurch freigelegten Klebefilme wird die Vorrichtung 1 auf der Hautoberfläche, beispielsweise einem Unterarm, einer Testperson befestigt. Anschließend wird die Abziehfolie 19 von der Abdeckeinheit 4 entfernt und letztere um im Wesentlichen 180° um das Gelenk 23 in die Rahmenöffnung 14 geklappt. Dabei sorgt der von der Abziehfolie 19 zuvor bedeckte Klebefilm an der Abdeckeinheit 4 für einen sicheren Halt derselben auf der Hautoberfläche. Alternativ ist denkbar, hier die Abziehfolie 19 einschließlich den von ihr abgedeckten Klebefilm an der Abdeckeinheit 4 ersatzlos weg zu lassen, da ein gewisser Halt der Vorrichtung 1 auf der Hautoberfläche bereits aufgrund der aufgeklebten Kennzeichnungseinheiten 6 gegeben ist. Nach dem Auflegen der Abdeckeinheit 4 auf die Haut erfolgt das Ritzen bzw. Pricken der einzelnen Allergieteststellen in derselben Weise, wie sie in Zusammenhang mit der ersten Ausführungsform beschrieben wurde. In Fig. 6 sind durch die Durchbrüche 5 hindurch die hier kreuzförmig ausgebildeten Durchgangslöcher 10 in der Abziehfolie 9 zu erkennen. Wie voranstehend beschrieben, wird dadurch die Zuverlässigkeit des Hautritzens im Kreuzschnittpunkt erfüllt.

Nach dem Ritzen der Haut wird die in Fig. 6 nicht zu erkennende Abziehfolie 9 abgezogen, so dass die Durchbrüche 5 vollständig freiliegen. Des Weiteren wird zum Öffnen der Behälter 3 die Schließfolie 18 von der Behältereinheit 2 entfernt. Daraufhin kann die Behältereinheit 2 mit den offenen Behältern 3 um im Wesentlichen 180° um das Gelenk 22 in die Rahmenöffnung 14 und damit auf die Abdeckeinheit 4 geklappt werden. Der von der Abziehfolie 9 zuvor bedeckte Klebefilm an der Abdeckeinheit 4 sorgt dabei für einen sicheren Halt der Behältereinheit 2 an der Abdeckeinheit 4. Es entsteht eine kompakt handhabbare Einheit umfassend das Rahmenteil 13, die Abdeckeinheit 4 und die Behältereinheit 2. Nach einer gewissen Einwirkdauer kann diese kompakt handhabbare Einheit vollständig von der Hautoberfläche entfernt werden, indem die Kennzeichnungseinheiten 6 niedergedrückt oder festgehalten werden und die kompakt handhabbare Einheit entlang der beiden perforierten Reißlinien 15 abgerissen wird.

### BEZUGSZEICHENLISTE

- 1: Vorrichtung
- 2: Behältereinheit
- 3: Behälter
- 4: Abdeckeinheit
- 5: Durchbruch
- 6: Kennzeichnungseinheit
- 7: Reißlinie
- 8: Schutzfolie
- 9: Abziehfolie
- 10: Durchgangslöcher
- 11: Kreismittelpunkt
- 12: Dichtlippe
- 13: Rahmenteil
- 14: Rahmenöffnung
- 15: Reißlinie
- 16: Gelenk
- 17: Schwammgewebe
- 18: Schließfolie
- 19: Abziehfolie
- 20: Durchgangsöffnungen
- 21: Bereich der Abziehfolie 9
- 22, 23: Gelenk

## Patentansprüche

1. Vorrichtung zur Durchführung eines Allergietests, umfassend eine Behältereinheit (2) mit mehreren Behältern (3) zur Aufnahme von Allergenen und eine Abdeckeinheit (4) mit mehreren Durchbrüchen (5) zur Abgrenzung von Allergieteststellen, wobei die Abdeckeinheit (4) auf die Haut eines Testlebewesens auflegbar ist und die Behältereinheit (2) derart auf die Abdeckeinheit (4) auflegbar ist, dass die Durchbrüche (5) mit den Behältern (3) im Wesentlichen fluchten,
**dadurch gekennzeichnet, dass**
auf derjenigen Seite der Abdeckeinheit (4), auf die die Behältereinheit (2) auflegbar ist, zumindest teilweise ein Klebefilm vorgesehen ist, der von einer Abziehfolie (9) bedeckt ist, die mit den Durchbrüchen (5) im Wesentlichen fluchtende Durchgangslöcher (10) aufweist.

2. Vorrichtung nach Anspruch 1,
**dadurch gekennzeichnet, dass**
die Querschnitte der Durchgangslöcher (10) vollständig innerhalb der Querschnitte der Durchbrüche (5) liegen.

3. Vorrichtung nach Anspruch 1 oder 2,
**dadurch gekennzeichnet, dass**
die Durchgangslöcher (10) und die Durchbrüche (5) dieselbe Querschnittsform und denselben Querschnittsmittelpunkt (11) aufweisen.

4. Vorrichtung nach Anspruch 2 oder 3,
**dadurch gekennzeichnet, dass**
die Querschnittsflächen der Durchgangslöcher (10) kleiner als die Querschnittsflächen der Durchbrüche (5) sind.

5. Vorrichtung nach einem der vorangehenden Ansprüche,
**dadurch gekennzeichnet, dass**
die Querschnitte der Durchgangslöcher (10) und/oder der Durchbrüche (5) Kreisform aufweisen.

6. Vorrichtung nach einem der vorangehenden Ansprüche,
**dadurch gekennzeichnet, dass**
die Querschnitte der Durchgangslöcher (10) und/oder der Durchbrüche (5) Rechteck-oder Quadratform aufweisen.

7. Vorrichtung nach einem der vorangehenden Ansprüche,
**dadurch gekennzeichnet, dass**
die Querschnitte der Durchgangslöcher (10) und/oder der Durchbrüche (5) Kreuzform aufweisen.

8. Vorrichtung nach einem der vorangehenden Ansprüche,
**dadurch gekennzeichnet, dass**
jeder Behälter (3) in seinem Randbereich eine Dichtlippe (12) aufweist.

9. Vorrichtung nach Anspruch 8,
**dadurch gekennzeichnet, dass**
jede Dichtlippe (12) bei auf die Abdeckeinheit (4) aufgelegter Behältereinheit (2) den jeweiligen Durchbruch (5) durchgreift, so dass sie mit der Haut um die jeweilige Allergieteststelle herum in Berührung gebracht werden kann und **dadurch** eine Abdichtung gegenüber benachbarten Allergieteststellen bewirkt wird.

10. Vorrichtung nach einem der vorangehenden Ansprüche,
**dadurch gekennzeichnet, dass**
sie wenigstens eine Kennzeichnungseinheit (6) zur Zuordnung von bestimmten Allergenen zu bestimmten Allergieteststellen auf der Haut des Testlebewesens aufweist, wobei die Kennzeichnungseinheit (6) lösbar an der Vorrichtung befestigt und auf ihrer bei Durchführung des Allergietests der Haut zugewandten Seite zumindest teilweise mit einem Haftmittel versehen ist, so dass die Vorrichtung bei auf der Haut verbleibender Kennzeichnungseinheit (6) entfernbar ist.

11. Vorrichtung nach Anspruch 10,
**dadurch gekennzeichnet, dass**
die Kennzeichnungseinheit (6) lösbar an einer Seitenkante der Behältereinheit (2) oder der Abdeckeinheit (4) befestigt ist, so dass die Behältereinheit (2) und die Abdeckeinheit (4) bei auf der Haut verbleibender Kennzeichnungseinheit (6) entfernbar sind.

12. Vorrichtu ng nach Anspruch 11,
**dadurch gekennzeichnet, dass**
die wenigstens eine Kennzeichnungseinheit (6) entlang einer perforierten Reißlinie (7) an der Behältereinheit (2) oder der Abdeckeinheit (4) befestigt ist.

13. Vorrichtung nach einem der Ansprüche 10 bis 12,
**dadurch gekennzeichnet, dass**
die wenigstens eine Kennzeichnungseinheit (6) um im Wesentlichen 180 zwischen einer ersten Stellung, in der sie auf der Behältereinheit (2) oder der Abdeckeinheit (4) aufliegt, und einer zweiten Stellung, in der sie von der Seitenkante der Behältereinheit (2) oder der Abdeckeinheit (4) abragt, schwenkbar ist.

14. Vorrichtung nach einem der Ansprüche 10 bis 13,
**dadurch gekennzeichnet, dass**
an zwei gegenüberliegenden Seitenkanten der Behältereinheit (2) oder der Abdeckeinheit (4) jeweils eine Kennzeichnungseinheit (6) angeordnet ist.

15. Vorrichtung nach einem der Ansprüche 10 bis 14,
**dadurch gekennzeichnet, dass**
die Behälter (3) aus der Behältereinheit (2) herauslösbar sind, so dass die Behältereinheit (2) oder die Behältereinheit (2) und die Abdeckeinheit (4) bei auf der Haut verbleibenden Behältern (3) entfernbar ist.

16. Vorrichtung nach einem der vorangehenden Ansprüche 10 bis 15,
**dadurch gekennzeichnet, dass**
ein Rahmenteil (13) mit einer Rahmenöffnung (14) vorgesehen ist, wobei die Behältereinheit (2), die Abdeckeinheit (4) und die wenigstens eine Kennzeichnungseinheit (6) jeweils an einer äußeren Seitenkante des Rahmenteils (13) befestigt sind.

17. Vorrichtung nach Anspruch 16,
**dadurch gekennzeichnet, dass**
die Behältereinheit (2) und die Abdeckeinheit (4) gelenkig an dem Rahmenteil (13) befestigt sind, so dass sie um im wesentlichen 180 in die Rahmenöffnung (14) klappbar sind.

18. Vorrichtung nach Anspruch 16 oder 17,
**dadurch gekennzeichnet, dass**
die wenigstens eine Kennzeichnungseinheit (6) entlang einer perforierten Reißlinie (15) an dem Rahmenteil (13) befestigt ist.

19. Vorrichtung nach einem der Ansprüche 16 bis 18,
**dadurch gekennzeichnet, dass**
die Behältereinheit (2) und die Abdeckeinheit (4) an gegenüberliegenden Seiten des Rahmenteils (13) befestigt sind.

20. Vorrichtung nach einem der Ansprüche 16 bis 19,
**dadurch gekennzeichnet, dass**
die wenigstens eine Kennzeichnungseinheit (6) um im Wesentlichen 180° zwischen einer ersten Stellung, in der sie im Wesentlichen in der Rahmenöffnung (14) liegt, und einer zweiten Stellung, in der sie von der Seitenkante des Rahmenteils (13) abragt, schwenkbar ist.

21. Vorrichtung nach einem der Ansprüche 16 bis 20,
**dadurch gekennzeichnet, dass**
an zwei gegenüberliegenden Seitenkanten des Rahmenteils (13) jeweils eine Kennzeichnungseinheit (6) angeordnet ist.

22. Vorrichtung nach einem der Ansprüche 10 bis 21,
**dadurch gekennzeichnet, dass**
das Haftmittel ein von einer entfernbaren Schutzfolie (8) bedeckter Klebefilm ist.

## Claims

1. A device for performing an allergy test, comprising a container unit (2) with several containers (3) for receiving allergens, and a cover unit (4) with several apertures (5) for demarcating allergy test sites, the cover unit (4) being able to be placed onto the skin of a living being undergoing a test, and the container unit (2) being able to be placed onto the cover unit (4) in such a way that the apertures (5) are substantially in alignment with the containers (3),
**characterized in that**
the side of the cover unit (4) onto which the container unit (2) can be placed is at least partially provided with an adhesive film, the latter being covered by a peel-off sheet (9) that has through-holes (10) substantially in alignment with the apertures (5).

2. The device as claimed in claim 1,
**characterized in that**
the cross sections of the through-holes (10) lie completely within the cross sections of the apertures (5).

3. The device as claimed in claim 1 or 2,
**characterized in that**
the through-holes (10) and the apertures (5) have the same cross-sectional shape and the same cross-sectional midpoint (11).

4. The device as claimed in claim 2 or 3,
**characterized in that**
the cross-sectional surface areas of the through-holes (10) are smaller than the cross-sectional surface areas of the apertures (5).

5. The device as claimed in one of the preceding claims,
**characterized in that**
the cross sections of the through-holes (10) and/or of the apertures (5) have a circular shape.

6. The device as claimed in one of the preceding claims,
**characterized in that**
the cross sections of the through-holes (10) and/or of the apertures (5) have a rectangular or square shape.

7. The device as claimed in one of the preceding claims,
**characterized in that**
the cross sections of the through-holes (10) and/or of the apertures (5) have a cross shape.

8. The device as claimed in one of the preceding claims,
**characterized in that**
each container (3) has a sealing lip (12) in its edge area.

9. The device as claimed in claim 8,
**characterized in that**
when the container unit (2) is placed onto the cover unit (4), each sealing lip (12) engages through the respective aperture (5) such that it can be brought into contact with the skin around the respective allergy test site and, in this way, provides a seal in relation to adjacent allergy test sites.

10. The device as claimed in one of the preceding claims,
**characterized in that**
at least one marking unit (6) for the purpose of allocating specific allergens to specific allergy test sites on the skin of the living being undergoing a test is provided, the marking unit (6) being secured releasably to the device and its side facing toward the skin during the allergy test is at least partially provided with an adhesive, such that the device can be removed while the marking unit (6) remains on the skin.

11. The device as claimed in claim 10,
**characterized in that**
the marking unit (6) is secured releasably to a side edge of the container unit (2) or of the cover unit (4), such that the container unit (2) and the cover unit (4) can be removed while the marking unit (6) remains on the skin.

12. The device as claimed in claim 11,
**characterized in that**
the at least one marking unit (6) is secured to the container unit (2) or the cover unit (4) along a perforated tear line (7).

13. The device as claimed in one of claims 10 to 12,
**characterized in that**
the at least one marking unit (6) can be pivoted through substantially 180° between a first position, in which it lies on the container unit (2) or the cover unit (4), and a second position, in which it protrudes from the side edge of the container unit (2) or of the cover unit (4).

14. The device as claimed in one of claims 10 to 13,
**characterized in that**
there is a marking unit (6) arranged on each of two opposite side edges of the container unit (2) or of the cover unit (4).

15. The device as claimed in one of claims 10 to 14,
**characterized in that**
the containers (3) are releasable from the container unit (2), such that the container unit (2) or the container unit (2) and the cover unit (4) can be removed while the containers (3) remain on the skin.

16. The device as claimed in one of the preceding claims 10 to 15,
**characterized in that**
a frame part (13) with a frame opening (14) is provided, and the container unit (2), the cover unit (4) and the at least one marking unit (6) are each secured on an outer side edge of the frame part (13).

17. The device as claimed in claim 16,
**characterized in that**
the container unit (2) and the cover unit (4) are secured to the frame part (13) in an articulated manner, such that they can be folded through substantially 180° into the frame opening (14).

18. The device as claimed in claim 16 or 17,
**characterized in that**
the at least one marking unit (6) is secured to the frame part (13) along a perforated tear line (15).

19. The device as claimed in one of claims 16 to 18,
**characterized in that**
the container unit (2) and the cover unit (4) are secured to opposite sides of the frame part (13).

20. The device as claimed in one of claims 16 to 19,
**characterized in that**
the at least one marking unit (6) can be pivoted through substantially 180° between a first position, in which it lies substantially in the frame opening (14), and a second position, in which it protrudes from the side edge of the frame part (13).

21. The device as claimed in one of claims 16 to 20,
**characterized in that**
there is a marking unit (6) arranged on each of two opposite side edges of the frame part (13).

22. The device as claimed in one of claims 10 to 21,
**characterized in that**
the adhesive is an adhesive film covered by a removable protective sheet (8).

## Revendications

1. Dispositif permettant d'effectuer un test d'allergie, comportant une unité de récipients (2) avec plusieurs récipients (3) servant à recevoir des allergènes et une unité de recouvrement (4) présentant plusieurs ouvertures (5) servant à délimiter des zones de tests d'allergies, sachant que l'unité de recouvrement (4) peut être posée sur la peau d'un cobaye et que l'unité de récipients (2) peut être posée sur l'unité de recouvrement (4) de telle sorte que les ouvertures soient globalement alignées avec les récipients (3),
**caractérisé en ce que**
sur le côté de l'unité de recouvrement (4) sur lequel peut être posée l'unité de récipients (2), un film adhésif est au moins partiellement prévu, lequel est recouvert d'un film plastique amovible (9) qui présente des trous de passage (10) globalement alignés avec les ouvertures (5).

2. Dispositif selon la revendication 1,
**caractérisé en ce que**
les sections transversales des trous de passage (10) reposent entièrement à l'intérieur de la section transversale des ouvertures (5).

3. Dispositif selon la revendication 1 ou 2,
**caractérisé en ce que**
les trous de passage (10) et les ouvertures (5) présentent la même forme de coupe transversale et le même centre de section transversale (11).

4. Dispositif selon la revendication 2 ou 3,
**caractérisé en ce que**
les surfaces des sections transversales des trous de passage (10) sont plus petites que les surfaces des sections transversales des ouvertures 5).

5. Dispositif selon l'une des revendications précédentes
**caractérisé en ce que**
les sections transversales des trous de passage (10) et/ou des ouvertures (5) présentent une forme circulaire.

6. Dispositif selon l'une des revendications précédentes
**caractérisé en ce que**
les sections transversales des trous de passage (10) et/ou des ouvertures (5) présentent une forme rectangulaire ou carrée

7. Dispositif selon l'une des revendications précédentes
**caractérisé en ce que**
les sections transversales des trous de passage (10) et/ou des ouvertures (5) sont cruciformes

8. Dispositif selon l'une des revendications précédentes
**caractérisé en ce que**
chaque récipient (3) est bordé d'une lèvre d'étanchéité (12)

9. Dispositif selon la revendication 8
**caractérisé en ce que**
chaque lèvre d'étanchéité (12) pénètre dans l'ouverture (5) respective lorsque l'unité de récipients (2) est posée sur l'unité de recouvrement (4), de telle sorte qu'elles puissent entrer en contact avec la peau autour de la zone de test d'allergies respective et forment ainsi une bague étanche aux zones de test d'allergies contigües.

10. Dispositif selon l'une des revendications précédentes
**caractérisé en ce que**
il présente au moins une unité d'identification (6) servant a classer certains allergènes avec certaines zones de test d'allergies sur la peau du cobaye, sachant que l'unité d'identification (6) est fixée au dispositif de façon amovible et qu'elle est équipée du moins partiellement d'un agent adhésif sur son côté orienté vers la peau lors de l'exécution du test d'allergies, de telle sorte que le dispositif puisse être retiré alors que l'unité d'identification (6) reste sur la peau

11. Dispositif selon la revendication 10,
**caractérisé en ce que**
l'unité d'identification (6) est fixée de façon amovible sur un bord latéral de l'unité de récipients (2) ou de l'unité de recouvrement (4), de telle sorte que l'unité de récipients (2) et l'unité de recouvrement (4) peuvent être retirées lorsque l'unité d'identification reste sur la peau (6).

12. Dispositif selon la revendication 11,
**caractérisé en ce que**
l'unité d'identification (6) au moins au nombre de une est fixée à l'unité de récipients (2) ou à l'unité de recouvrement (4) le long d'une ligne de déchirement perforée (7).

13. Dispositif selon l'une des revendications 10 à 12,
**caractérisé en ce que**
l'unité d'identification (6), au moins au nombre de une, pivote d'au moins 180° entre une première position dans laquelle elle repose sur l'unité de récipients (2) ou l'unité de recouvrement (4) et une seconde position dans laquelle elle dépasse du bord latéral de l'unité de récipients (2) ou de l'unité de recouvrement (4).

14. Dispositif selon l'une des revendications 10 à 13
**caractérisé en ce que**
sur chacun des deux bords latéraux opposés de l'unité de récipients (2) ou de l'unité de recouvrement (4) est disposée une unité d'identification (6).

15. Dispositif selon l'une des revendications 10 à 14
**caractérisé en ce que**
les récipients (3) peuvent être retirés de l'unité de récipients (2) de telle sorte que l'unité de récipients (2) ou l'unité de récipients (2) et l'unité de recouvrement (4) puissent être retirées alors que les récipients (3) restent sur la peau.

16. Dispositif selon l'une des revendications 10 à 15
**caractérisé en ce que**
un cadre (13) avec une ouverture (14) est prévu, sachant que l'unité de récipients (2), l'unité de recouvrement (4) et l'unité d'identification (6) au moins au nombre de une, sont chacune fixées à un bord latéral extérieur du cadre (13).

17. Dispositif selon la revendication 16
**caractérisé en ce que**
l'unité de récipients (2) et l'unité de recouvrement (4) sont fixées au cadre (13) de façon articulée, de telle sorte qu'elles puissent être repliées globalement à 180° dans l'ouverture du cadre (14).

18. Dispositif selon la revendication 16 ou 17
**caractérisé en ce que**
l'unité d'identification (6) au moins au nombre de une, est fixée au cadre (13) le long d'une ligne de déchirement perforée (15).

19. Dispositif selon l'une des revendications 16 à 18
**caractérisé en ce que**
l'unité de récipients (2) et l'unité de recouvrement (4) sont fixées sur des côtés opposés du cadre (13).

20. Dispositif selon l'une des revendications 16 à 19
**caractérisé en ce que**
l'unité d'identification (6) au moins au nombre de une, pivote globalement à 180° entre une première position dans laquelle elle repose globalement dans l'ouverture du cadre (14) et une seconde position dans laquelle elle dépasse du bord latéral du cadre (13).

21. Dispositif selon l'une des revendications 16 à 20
**caractérisé en ce que**
sur les deux bords latéraux opposés du cadre (13), est respectivement disposée une unité d'identification (6).

22. Dispositif selon l'une des revendications 10 à 21
**caractérisé en ce que**
l'agent adhésif est un film adhésif recouvert d'un film protecteur (8) amovible.
